# EUROPEAN PATENT APPLICATION

(11) **EP 0 564 392 A2**
(43) Date of publication of application: **06.10.1993**
(21) Application number: 93500026.5
(22) Date of filing: 03.03.1993
(51) Int. Cl.: A61B 17/54, A61B 19/00, B24C 7/00

(54) **Medical equipment, useful in the cutaneous dermabrasion technique, achieved by means of abrasive powder**

(30) Priority: 04.03.1992 ES 9200486; 18.03.1992 ES 9200599; 28.10.1992 ES 9202167
(71) Applicant: Fructuoso Martinez, D. Antonio, E-30008 Murcia (ES)
(72) Inventor: Fructuoso Martinez, D. Antonio, E-30008 Murcia (ES)
(74) Representative: Blumbach, Kramer & Partner

(57) **Abstract**

The invention relates to an equipment useful in the cutaneous dermabrasion technique, using abrasive powder, comprising three groups of components; one being an application component (1), another being a pressure component (2) and another a suction component (3), all coordinated with each other and the first comprising a head (10), a middle part (11), an abrasive powder delivery conduit (12) and a used powder collecting conduit (13), the pressure component comprising a compressor (4), a reserve air tank (5) and an abrasive powder tank (6), and the suction component comprising a vacuum pump (7), a filters container (9) and a used powder collecting tank (8). Several embodiments are described for some of the elements, in an attempt at enhancing the effects thereof.

## Description

The invention relates as set out in the heading to an equipment that can be used in the cutaneous dermabrasion technique, which has been conceived with a view to being applied in medicine, making it possible to use the technique with abrasive powder, such as corundum, to achieve precise and satisfactory results, given that such dermabrasion is easy to control as regards extent and depth.

The description of the working equipment, designed for skin treatment, will be illustrated with a set of drawings showing a preferred embodiment of the invention, which is not restrictive but merely illustrative and may hence be subject to detail variations of whatever is not an essential alteration of the characteristic aim thereof.

In the drawings:

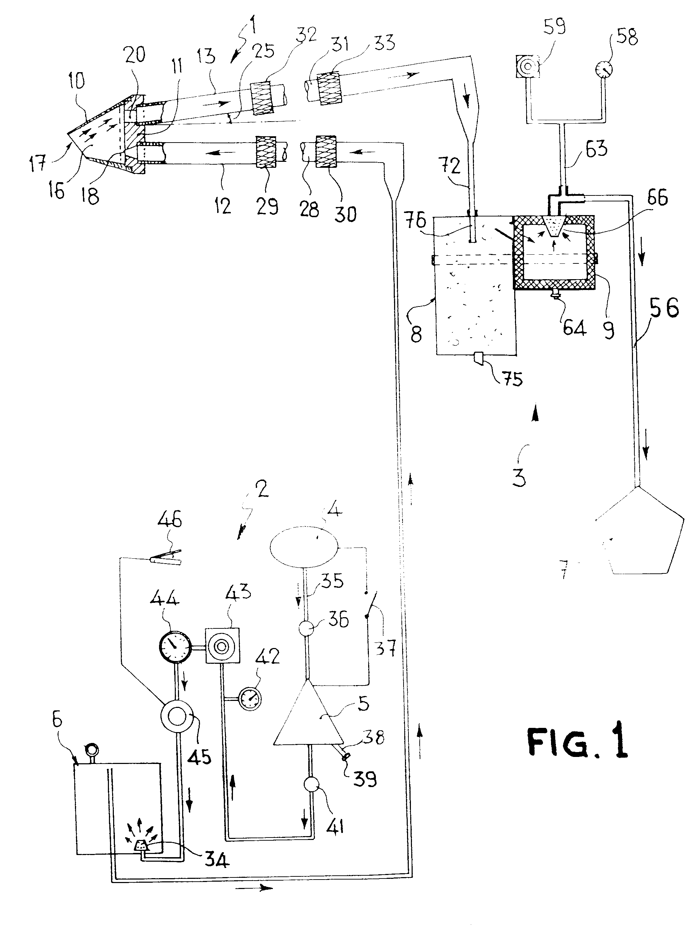

Figure 1 is a diagrammatic view of the equipment subject of the invention in its entirety.

Figure 2 is an enlarged detail of the part of the equipment corresponding to the application component.

Figure 3 is an enlarged detail of the part of the equipment corresponding to the pressure component.

Figure 4 is an enlarged detail of the part of the equipment corresponding to the suction component.

Figure 5 is an improved embodiment of the suction mechanism. (Patent no. 9200599 Priority 18-3-92).

Figure 6 is a diagrammatic view of the same medical equipment in its entirety, with improvements in some of its elements. (Patent no. 9202167 Priority 28-10-92).

Figure 7 is an enlarged detail of the filter and powder and detritus collecting tank container in a different embodiment. (Patent no. 9202167 Priority 28-10-92).

Number references have been established in the drawings to designate the various parts making up each component as a whole.

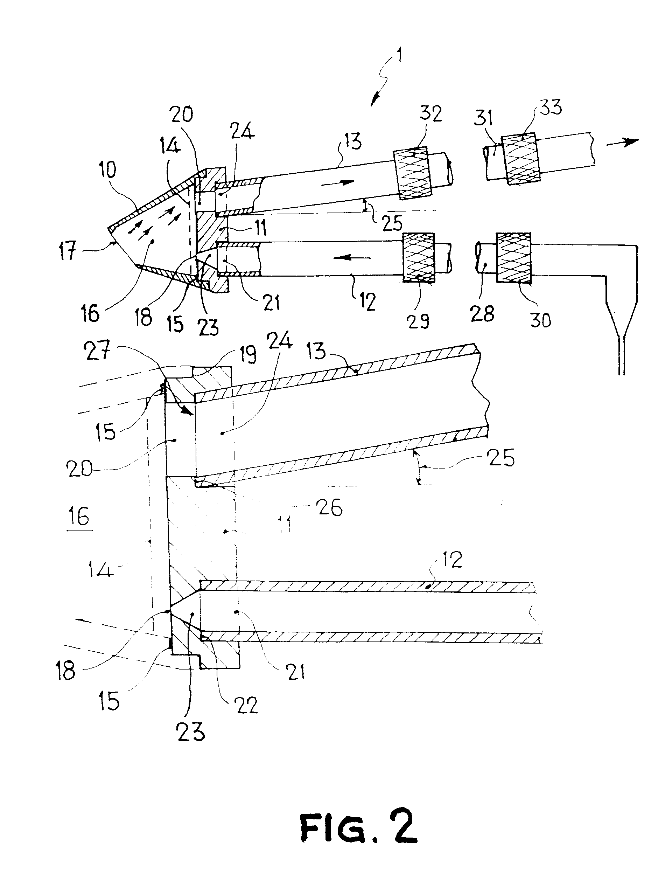

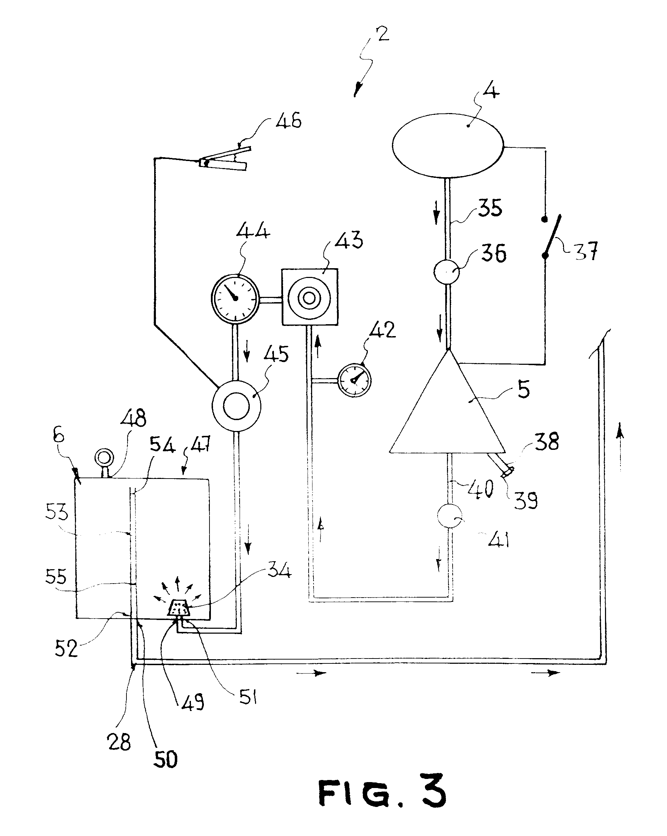

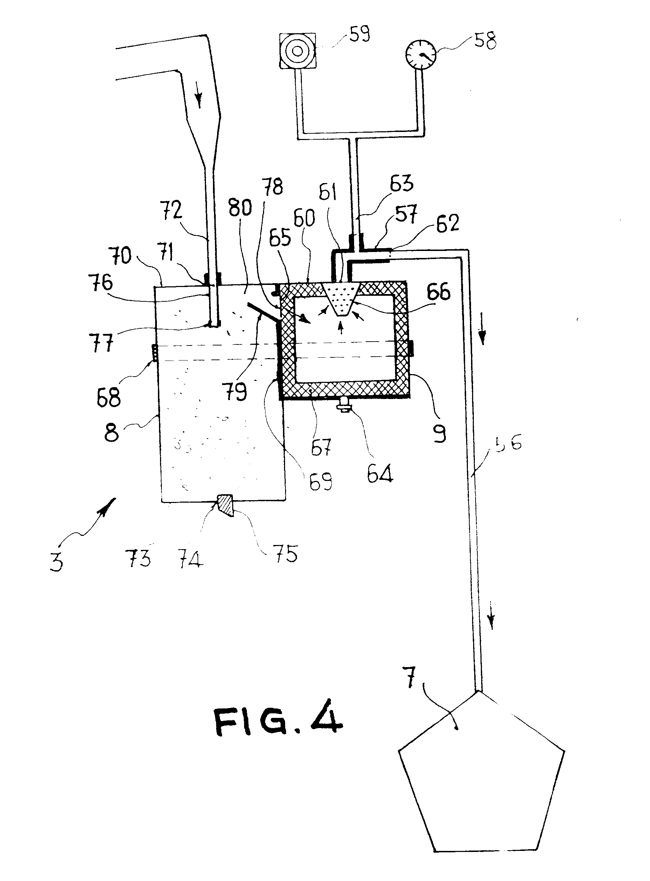

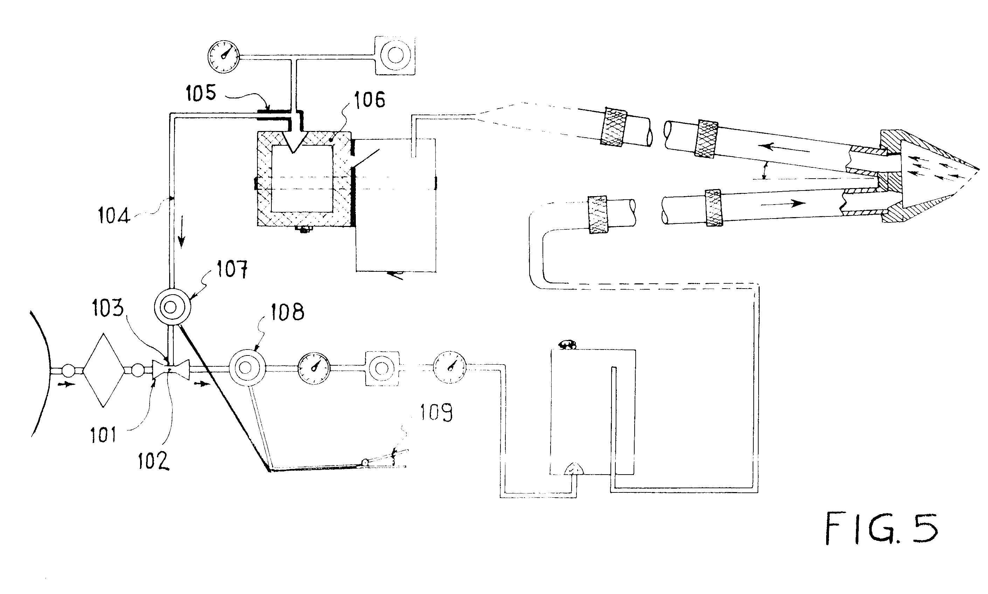

With reference to the drawings, the medical equipment subject of the invention is based upon the coordination of its application component (figure 2), a pressure mechanism (figure 3) and a suction mechanism (figure 4).

### Application component.

This component is made of stainless steel material as used for surgical materials, and its parts are joined to each other by a metallurgical sealing technique, thereby conferring the same the property to withstand high temperature and to be watertight (having no kind of elastic joint, for it would lose its ability to withstand high temperatures).

The component can, because of its manufacturing characteristic, be sterilised entirely, thereby to avoid interpersonal contagion, for the dermabrasion technique breaks the defensive cutaneous barrier on penetrating the skin. (If the materials should have an elastic joint or a joint not withstanding high temperatures, the equipment could not be sterilised, and interpersonal contagion would ensue).

The application component (figure 2) comprises four essential parts: a head (10); a middle part (11); an abrasive powder delivery unit (12); an abrasive powder and detritus suction or collecting conduit (13).

a). Head (10). The head is hollow, frustum-shaped and with a circular and open larger base (14), with a notching on its inner face (15) located next to the side edge, wherein is fitted the middle part (11), which communicates with the inner space of the hollow (16) in the head through two holes (18-20).

The smaller base is oblique and corresponds with the output hole (17); its centre forms a horizontal axis with the output hole (18) for the abrasive powder of the middle part, the head part being what is placed in contact with the skin.

b). Middle part (11). This middle part is cylindrical in shape, with a side, inner and outer face. The side face is circular and has a notching on the inner edge (19) which is coupled with the notching on the larger base of the head (14).

The inner face is flat and has two holes (18-20), the inner hole (18) corresponding with the abrasive powder output, being four times smaller in size than the upper hole (20). The upper hole (20) corresponds with the suction component (figure 4) and is four times larger than the abrasive powder output hole (18).

The efficiency of the application component is not altered by other possible changes between the holes, provided that the lower hole (18) has a diameter ranging between 0.8 mm and 3 mm, and the suction hole has a diameter lying between 3 mm and 15 mm, subject that the suction hole (20) is equal to or greater than the abrasive powder output hole (18).

It has at the same time been shown that no efficiency is lost if metallic sealing is not effected to join its four components, provided that a hermetic adjustment system is used for the head (10) with the middle part (11) and a thread system between the middle part (11) and both metallic conduits (12 and 13). This modification makes it easier to clean and sterilise its four components separately. (Patent 9202167 Priority 28-10-92).

The outer face is flat and provided with two holes, the inner hole (21) being provided on its rim with a notching (22) wherein is coupled the metallic abrasive powder delivery conduit (12).

The inner holes of the outer (21) and inner (18) faces communicate with each other through a cup-shaped conduit (23), whose vertex corresponds with the inner face hole (18) and the base the external face (21); thus, the abrasive powder is directed without finding any obstacles on its path.

The upper hole (24) is oblique in direction, forming an angle (25) of between twenty and thirty-five degrees. It has a notching (26) of equal angle, expediting coupling of the metallic suction conduit (13) and communicates with the inner hole (20) by means of a cylindrical conduit (27).

It can be observed that this part (11) communicates the outside with the head (17) and the abrasive powder delivery (12) and collecting (13) (suction) conduits.

c). Powder delivery conduit (12). It is horizontally disposed and coupled through an end to the notching (22) in the outer lower hole (21) of the middle part (11), its centre axis horizontally coinciding with the centre of the output hole of the head (17), and connected through its other end to the non-metallic conduit (28) leading from the tank (6) of the abrasive powder to be used. This connection is improved with a thread system, one of its components (29) lying five millimetres away from the joint of the metallic conduit (12) with the non-metallic conduit (28), leading from the tank (6) of the abrasive powder to be used.

d). Suction or powder collecting conduit (13). This conduit is coupled at the notching (26) in the upper outer hole of the middle part (11) forming an angle (25) to the powder delivery conduit, of between twenty and thirty-five degrees. The diameter of this conduit is two or three times greater than that of the abrasive powder delivery conduit.

At the opposite end it connects with the non-metallic conduit (31) leading from the abrasive powder and detritus collecting tank (8), which connection is reinforced with a thread system, having a component (32) five millimetres away from the joint of the metallic conduit (13) with the non-metallic conduit (31) and the other component (33), in the non-metallic conduit (31), leading from the abrasive powder and detritus collecting tank (8).

### Pressure component.

The pressure of this component is to allow the abrasive powder to have an impact power on the skin, notoriously greater than the impact caused by the suction mechanism.

This component essentially comprises: a) a compressor (4); b) a reserve air tank (5); a tank for the abrasive powder to be used (6).

a). Compressor (4). The compressor will preferably be an air compressor (oil compressors lose oil with on being used which is micronised and on reaching the tank (6) of the abrasive powder to be used form adherences in the air diffuser (34) located within the tank (6), diminishing the performance of the equipment) and communicating with the reserve air tank (5) through a conduit pipe (35), administering the air, a filter (36) being provided between the reserve air tank (5) and the compressor (4) to prevent humidity in the compressed air generated by the compressor (4), above all in places with a large amount of atmospheric humidity.

The compressor is provided with an electromechanical start-stop mechanism (37) which begins to operate when the preset air pressure in the reserve air tank (5) varies.

b). Reserve air tank (5). This tank holding the compressed air taken from the compressor (4), is provided at its output with two conduits, one (38) discharging manually to the outside for emptying the same, controlled by a manual opening-closing mechanism (39) and another conduit (40) having a second air filter (41) to avoid the rest of air humidity, if any.

This conduit communicates with an indicator (42) located on a control panel, which reflects the reserve air status (5), moreover communicating with a valve type control knob (43) and the latter in turn with an indicator (44) at which the working pressure can be fixed.

In order to have the air reach the abrasive powder tank (6), once the pressure has been fixed, the electromechanical control (45) is used, making it work with a foot-operated switch (46) located outside the equipment, which will on being pressed determine starting, whereupon the air will at the set pressure move to the abrasive powder tank (6).

When pressing is discontinued, stopping will come about and the delivery of air at the abrasive powder tank (6) will stop.

c). Tank of abrasive Powder to be used (6). Such is cylindrical in shape, its upper face (47) having an opening and hermetic closing hole (48) for filling with sterile abrasive powder.

The lower face is provided with two holes, one for the compressed air delivery conduit (49) leading from the pressure mechanism (2) and another conduit (50) for the abrasive powder output conduit (28) which will connect through a non-metallic conduit (27) with the metallic abrasive powder delivery conduit (12) of the application control (1). Its inside moreover has two holes, the first of which (51) communicates through a conduit with the compressed air delivery hole (49) and in which an air diffuser (34) is provided, causing the abrasive powder to be used to begin moving.

The second hole (52) communicates through a conduit with the outer hole (50) corresponding to the output conduit of the abrasive powder to be used.

The said hole is fitted with a vertical pipe (53) opening at its upper part (54) and having a hole (55) located on the lower quarter of the said pipe (53) through which the abrasive powder penetrates when it is moving, then going into the non-metallic abrasive powder output conduit (28).

### Suction component.

The suction component (figure 4) comprises: a vacuum pump (7); a filters container (9) and a used abrasive powder and detritus collecting tank (8).

a). Vacuum pump. The vacuum pump (7) communicates with the filter container (9) through a non-metallic conduit pipe (56) with a diameter of between two and twenty millimetres (the larger the size, the better the performance and the lesser the chances are of the application component being blocked).

The coupling system (57) of the filters container (9) communicates with an indicator (58) on the control panel, which gives the vacuum value and further communicates with a valve type control knob (59) with which the working vacuum value is fixed.

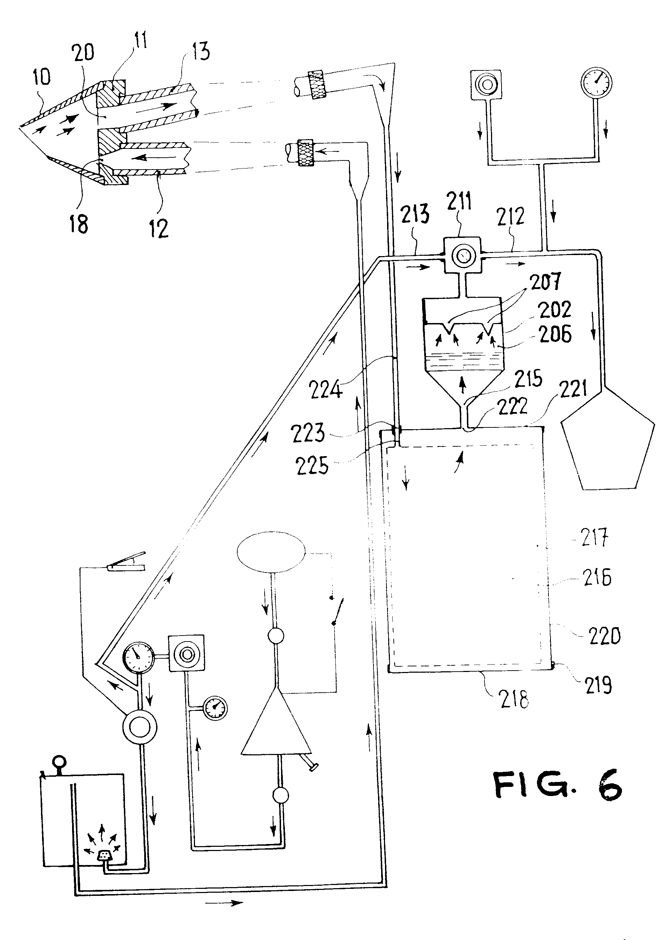

In an alternative embodiment of the invention, the vacuum pump is substituted (figure 6) with a Venturi type system (101) in which when the compressed air passes, a vacuum is formed in the narrower part (102) thereof, which is provided with a hole (103) which connects through a conduit (104) with the coupling system (105) of the filter container (106).

The equipment is controlled by means of an electromechanical or pneumatic valve mechanism (107) acting in synchrony with the electromechanical valve system (108) controlling the pressure mechanism, so that if one is activated the other remains idle and vice versa.

The said control is exercised by means of a switch type pedal (109) having three positions, a neutral position, at which the pressure and vacuum systems are both at rest, and two other positions, one actuating the pressure mechanism, at which the Venturi effect is idle, and the second one actuating the Venturi effect, with the pressure component remaining idle.

The power of the compressor must preferably range between 0.5 and 2 HP. (Patent 9200599 Priority 18-3-92).

b). Filters container (9).

It is cylindrical; its upper outer face (60) has a hole (61) leading into the same, and a system (57) is coupled therein having two openings, one (62) for the vacuum pump (7) and the other for the conduit pipe (63), from the control mechanism (59) (58).

The lower face is provided with an opening system (64) to expedite access to the filters system (9).

A part of the side face (65) communicates with the used abrasive powder and detritus collecting tank (8), expediting the functionality of the suction mechanism (3). Inside the filters container, located internally in the upper face, there is a metallic filter (66) and surrounding its entire inner side face another netting type (69) filter is provided, some two to three centimetres thick, whose purpose is to preserve the perfect functionality of the metallic filter (66), for if the latter is blocked, the suction mechanism (3) will be interrupted.

The filters container (9) is fixed to the tank (8) of abrasive powder to be used by a clamp (68), an elastic joint (69) being provided between both tanks which prevents the passage of air from the outside to the inside of the components, without altering the suction mechanism (3).

The filter container can be rendered more operative by introducing the following improvements:

A change in shape is introduced, providing the same with an upper cylindrical part (203), the lower part ending in a taper (204) with the vertex pointing down. The cylindrical part (203) is divided in to two compartments, an upper compartment (205) and a lower compartment (206), both having a hermetic seal and communicating with each other through one or several holes (207) in which one or several metallic filters (208) are coupled, depending on the number of holes (207).

The upper face of the upper cylindrical compartment (205) has a hole (210) with an output, wherein is coupled a valve control system (211) connecting through a path with the suction system (212) and through another with the pressure component (213). This valve system (211) carries out the cleaning function of the filter system (208 and 214) when the pressure system path is opened (213), compressed air passing through such system, the suction system being at the same time closed and hence suffering no damage whatsoever.

The valve system (211) can be of one of the following kinds:
a) Manual: a lever system is used to open a path to expedite the operation of a system, pressure or suction, the opposite one being closed.
b) Electromechanical: automatic activation by means of a timer or an electric switch.
c) By a pneumatic system.

The cylindrical portion (206) has the netting type filter (214) and the taper vertex (215) communicates (222) in filter system (202) with the powder and detritus collecting tank (216). (Patent 9202167 Priority 28-10-92).

c). Used abrasive powder and detritus collecting tank (8). Such comprises a cylindrical container with a hermetic seal whose upper face (70) has a hole (71) communicating with the inside of the container, in which the non-metallic conduit pipe (72) is connected, which communicates at the other end with the suction metallic conduit (13) of the application component (1).

The lower face (73) has a hole (74) communicating with the inside of the tank (8) to empty the same, being provided with a hermetic sealing system (75) in order that the suction component is not altered (3).

The inside of the container (8) coinciding with the upper face hole (71) is communicated by a small conduit pipe (76) with a length of some five centimetres and a free lower end (77), through which the now used abrasive powder and cutaneous detritus are dropped into the tank (8).

The side face in communication with the filters container (9) through a communication hole (78) has an angle sheet (79) which partially surrounds the said hole (78), its lower part having an upward direction and its free end (80) lying three centimetres from the upper face of the container (70).

Through this sheet (79) and the small conduit pipe (76) the delivery of particles of abrasive powder and cutaneous detritus at the filters system (9) is largely avoided. When the suction mechanism (3) is in operation, and the application knob input hole (17) remains free, the abrasive powder collecting tank (8) is provided with a current of upward air which drags particles up to the filters system (9).

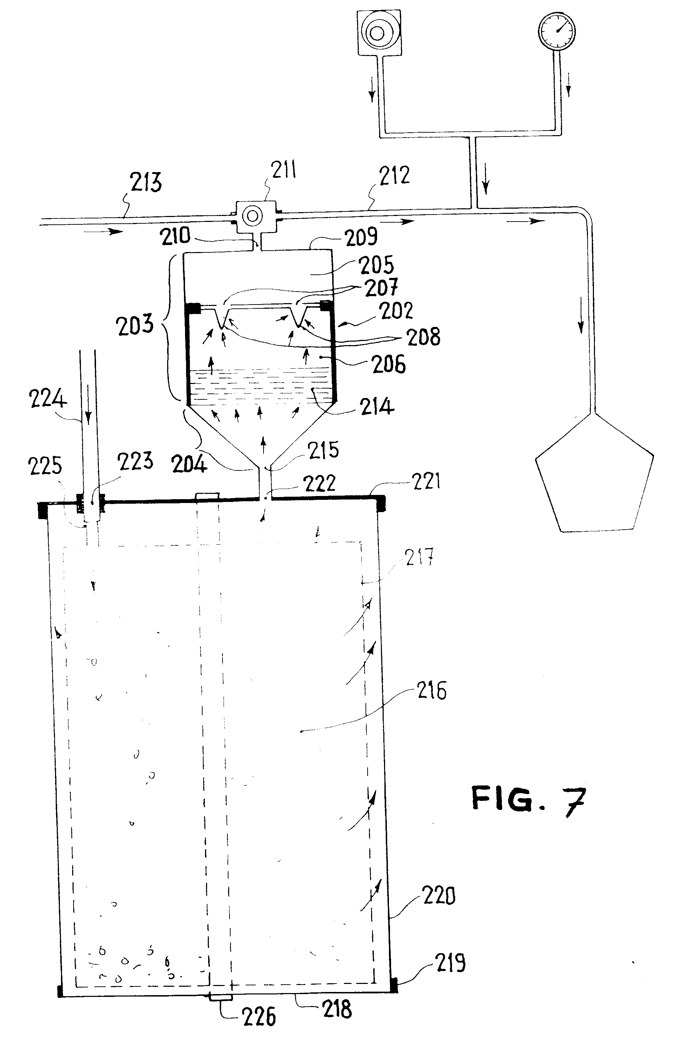

A different embodiment improving this tank (figures 6 and 7) essentially comprises coupling inside the said tank (216) a bag of porous material (217) with a three-fold purpose:
1.- To collect used powder and detritus.
2.- To act as a filter, protecting the suction component which it does not alter.
3.- To absolutely avoid contagion of the manipulator of used powder and detritus, which risk existed heretofore.

The material of which the bag (217) is made can be paper, cloth or any other kind of material allowing the passage of air and not of abrasive powder and detritus.

In order to allow the porous bag (217) to be coupled inside the powder and detritus collecting tank (216) the following changes have been made:
1.- The tank (216) is cylindrical with a flat lower face (218) and a hermetic seal (219) to the cylinder body (220).
2.- The upper face (221) is provided with two holes, one of which (222) communicates with the filter system (202) through the vertex of the taper hole (215) and another hole (223) through which the outer part of the non-metallic suction conduit (224) from the application component is connected.

The inner face of the said hole (223) connects with the connection conduit (225) of the porous bag (217). This porous bag (217) is smaller in size than the powder and detritus collecting tank (216).

The abrasive powder and detritus tank (216) is divided into two parts, the first being the lower face (218), joined (219) to the cylindrical body (220) and the second part being the upper face (221), thereby expediting manipulation to remove and place the said porous bag (217).

The seal of the parts of the abrasive powder and detritus tank (216) can be of a thread kind or using a clamp system (226) surrounding the said abrasive powder and detritus tank. (Patent 9202167 Priority 28-10-92).

After describing this invention in sufficient depth, the most important characteristics of the equipment are listed below.
1.- The equipment fulfils the medical principles of safe sterilisation, preventing interpersonal contagion.
2.- The said safety stems from the possibility of sterilising the application component (1) in its entirety.
3.- The equipment is preferably made of a stainless steel material, as in surgical material.
4.- The various components form an integrated unit, being metallurgically sealed, conferring the same a hermetic capacity.
5.- The angular arrangement (25) between the abrasive powder delivery conduits (12) and suction conduits (13) confer the same the ability to be separated from the rest of the equipment, without any tool whatsoever being required.
6.- The existing ratio between the powder output hole (18) of the middle part (11) and the powder collecting hole (20) is ideal to enhance the performance of the equipment and prevent the suction mechanism (3) from being blocked.
7.- The shape used for the head (10) of the application component is ideal to work in areas of difficult access, which would not be possible if a bell shape were used.
8.- The equipment's provision with a foot-operated switch (46) for the pressure component (2) expedites the manipulation thereof.
9.- The provision of the air compressor (4) prevents the air diffuser (34) in the tank of sterile abrasive powder to be used (6) from being blocked, enhancing the performance of the equipment.
   If the compressor (4) were an oil compressor, particles thereof would escape and block the diffuser (34), diminishing the performance and even causing the system to break by explosion of the powder containing tank (6).
10.- The existence of an abrasive powder and detritus collecting tank (8) independent of the filter container (9), implies that the suction power of the suction pump (7) will not be held back, increasing the performance power of the equipment.
11.- The presence of a small pipe (76) inside the powder tank (8) with a length of five centimetres prevents the particles of detritus and used abrasive powder from entering the filter container (9) due to the suction dragging force.
12.- The presence of the angle sheet (79) in the used powder collecting tank (8) forms a conduit (80) with the upper inner face and the wall of such container (8), such sheet acting as a barrier in the event of a particle rising due to the suction force.
13.- The elastic joint (69) located between the powder collecting tank (10) and the filters container (9) confers the assembly with a hermetic capacity that is improved with the external clamps system (68).
14.- The netting type filter (67) of the filter container (6) acts to protect the metallic filter (66), the latter remaining permeable and hence remaining in the optimal equipment position.
15.- The ease of access of the filters tank (9) expedites the manipulation thereof.
16.- The diameter of the conduit pipe (56) of the vacuum pump (7) to the coupling system in the filters container (57) is of twelve to twenty millimetres, whence the pump (7) suction capacity is notoriously increased.

It should finally be noted that all shape and functional conditions that do not alter the essence of the invention can be changed, and all should be included in the protection being requested.

## Claims

**1.-** MEDICAL EQUIPMENT, USEFUL IN THE CUTANEOUS DERMABRASION TECHNIQUE, ACHIEVED BY MEANS OF ABRASIVE POWDER, characterised in comprising three component groups, the first being the application component and the other two being the pressure and suction components, functionally coordinated with the first.

**2.-** MEDICAL EQUIPMENT, USEFUL IN THE CUTANEOUS DERMABRASION TECHNIQUE, ACHIEVED BY MEANS OF ABRASIVE POWDER, as in claim 1, characterised in that the application component comprises four essential parts: a head (10), a middle part (11), an abrasive powder delivery conduit (12) and an abrasive powder and detritus suction or collecting conduit (13), the head (10) being hollow, frustum-shaped, with a larger base (14) and a notching (15) on its inner face, wherein is fitted the middle part (11), leading into the hollow (16) of the head, through two holes (18-20), the lower base being oblique, its centre forming a horizontal axis with the abrasive powder output hole (18).

**3.-** MEDICAL EQUIPMENT, USEFUL IN THE CUTANEOUS DERMABRASION TECHNIQUE, ACHIEVED BY MEANS OF ABRASIVE POWDER, as in claims 1 and 2, characterised in that the middle part of the application component is cylindrical in shape, with a side, inner and outer face, the side face being circular, provided with a notching (19) on its inner edge, to which the notching of the larger base of the head (15) is coupled.

**4.-** MEDICAL EQUIPMENT, USEFUL IN THE CUTANEOUS DERMABRASION TECHNIQUE, ACHIEVED BY MEANS OF ABRASIVE POWDER, as in claim 3, characterised in that the inner face of the middle part is flat, with two holes (18-20), the inner hole (18) being four times smaller than the upper hole (20) corresponding to the abrasive powder output and the upper hole corresponding to the suction component.

**5.-** MEDICAL EQUIPMENT, USEFUL IN THE CUTANEOUS DERMABRASION TECHNIQUE, ACHIEVED BY MEANS OF ABRASIVE POWDER, as in claims 1 to 4, characterised in that the ratio between the lower abrasive powder output hole (18) and the suction hole (20) provided in the application component, forming part of the apparatus, can vary, provided that the former ranges between 0.8 mm and 3 mm in diameter and the latter between 3 mm and 15 mm in diameter, subject that the suction hole (20) is equal to or larger than the abrasive powder output hole (18) (Patent 9202167 Priority 28-10-92).

**6.-** MEDICAL EQUIPMENT, USEFUL IN THE CUTANEOUS DERMABRASION TECHNIQUE, ACHIEVED BY MEANS OF ABRASIVE POWDER, as in claims 1 to 5, characterised in that the union between the four main parts of the application component, namely head, middle part, abrasive powder delivery conduit and abrasive powder and detritus suction or collecting conduit, is achieved by means of a hermetic adjustment system of the head (10) and the middle part (11), and a thread system between the middle part (11) and both metallic conduits (12) and (13). (Patent 9202167 Priority 28-10-92).

**7.-** MEDICAL EQUIPMENT, USEFUL IN THE CUTANEOUS DERMABRASION TECHNIQUE, ACHIEVED BY MEANS OF ABRASIVE POWDER, as in claims 3 and 4, characterised in that the outer face of the middle part has two holes, the lower hole (21) being provided at its rim with a notching (22) wherein is coupled the metallic abrasive powder delivery conduit (12) and the upper hole being oblique in projection, provided with a notching (26) allowing the metallic suction conduit (13) to be coupled, and communicating with the inner hole (20) through a cylindrical conduit (27). The lower holes (21) on the outer face and (18) on the inner face communicate with each other through a cup-shaped conduit (23) whose vertex corresponds with the hole on the inner face (18) and the base with the outer face (21).

**8.-** MEDICAL EQUIPMENT, USEFUL IN THE CUTANEOUS DERMABRASION TECHNIQUE, ACHIEVED BY MEANS OF ABRASIVE POWDER, as in claims 1 and 2, characterised in that the powder delivery conduit (12) is arranged horizontally, being coupled at an end with the notching (22) in the hole (21) of the middle part (11), and at the other end with the conduit (28) leading from the tank (6) of the abrasive powder to be used, the said connection being improved with a thread system (29) (30).

**9.-** MEDICAL EQUIPMENT, USEFUL IN THE CUTANEOUS DERMABRASION TECHNIQUE, ACHIEVED BY MEANS OF ABRASIVE POWDER, as in claims 1 and 2, characterised in that the powder suction or collecting conduit (13) is coupled in the outer notching (26) of the upper hole of the middle part (11), its diameter being two or three times greater than that of the abrasive powder delivery conduit (28) and being coupled through the opposite end on the non-metallic conduit from the abrasive powder collecting tank (6) this connection being moreover reinforced through a thread system (32-33).

**10.-** MEDICAL EQUIPMENT, USEFUL IN THE CUTANEOUS DERMABRASION TECHNIQUE, ACHIEVED BY MEANS OF ABRASIVE POWDER, as in claim 1, characterised in that the pressure component (2), functioning to allow the abrasive powder to have an impact on the skin greater than that caused by the suction abrasive component (3), essentially comprises a compressor (4), a reserve air tank (5) and a tank for the abrasive powder to be used (6), the compressor being preferably an air compressor which communicates with the reserve air tank (5) through the conduit pipe (35), there being between the reserve air tank (5) and the compressor (4) a filter (36) to avoid humidity in the compressed air. The compressor also has an electromechanical start-stop mechanism (37) which begins to work when the preset air pressure in the reserve air tank varies.

**11.-** MEDICAL EQUIPMENT, USEFUL IN THE CUTANEOUS DERMABRASION TECHNIQUE, ACHIEVED BY MEANS OF ABRASIVE POWDER, as in claims 1 and 8, characterised in that the reserve air tank (5) holding the compressed air from the compressor (4) is provided at its output with two conduits, one discharging manually (38) to the outside, controlled by an opening-closing mechanism (39), and another one having a second air filter (41) to avoid the rest of air humidity, communicating with an indicator (42) located on the control panel, which reflects the position of the reserve air (5) and on its other side with a valve control knob (43), and the latter in turn with an indicator (44), where the working pressure is fixed.

**12.-** MEDICAL EQUIPMENT, USEFUL IN THE CUTANEOUS DERMABRASION TECHNIQUE, ACHIEVED BY MEANS OF ABRASIVE POWDER, as in claims 1, 8 and 9, characterised in that the reserve air tank is fitted with an electromechanical control (45) activated by a foot switch (46) which regulates the passage of air into the abrasive powder tank (6) when it is started and cuts the same when it is stopped.

**13.-** MEDICAL EQUIPMENT, USEFUL IN THE CUTANEOUS DERMABRASION TECHNIQUE, ACHIEVED BY MEANS OF ABRASIVE POWDER, as in claims 1 and 8, characterised in that the tank of abrasive powder to be used (6) is cylindrical and is fitted on its upper face with a hole (48) opening and closing hermetically for filling the sterile abrasive powder, its lower face having two holes, a hole (49) for the compressed air delivery conduit, from the pressure mechanism, and another hole (50) for the abrasive powder output conduits, connecting through the conduit (28) with the abrasive powder delivery conduit (12). The inside of the tank is also provided with two holes, the first (51) communicating through a conduit with the compressed air delivery hole (49) and an air diffuser (34) causing the abrasive powder to move, and the second hole communicating through a conduit with the outer hole (50) in the powder output conduit, the said hole being vertically provided with a pipe, open at its upper end (54) with a hole (55) on its lower part, through which abrasive powder enters, thereafter to move to the output conduit (28).

**14.-** MEDICAL EQUIPMENT, USEFUL IN THE CUTANEOUS DERMABRASION TECHNIQUE, ACHIEVED BY MEANS OF ABRASIVE POWDER, as in claim 1, characterised in that the suction component essentially comprises a vacuum pump (7), a filter container (9), and a tank (8) for collecting used abrasive powder and detritus, the vacuum pump communicating with the filter container (9) through a non-metallic pipe (56), the coupling system (57) of the filter container (9) communicating with an indicator (58) on the control panel indicating the vacuum value and on the other hand with a valve type control knob (59) with which the working vacuum value is fixed.

**15.-** MEDICAL EQUIPMENT, USEFUL IN THE CUTANEOUS DERMABRASION TECHNIQUE, ACHIEVED BY MEANS OF ABRASIVE POWDER, as in claims 1 to 14, characterised in that the vacuum pump forming part of the apparatus' suction component is substituted by a Venturi system (101) whose narrowest part (102) is provided with a hole (103) connecting through the conduit (104) with the coupling system (105) of the filter container (106). (Patent 9200599 Priority 18-3-92).

**16.-** MEDICAL EQUIPMENT, USEFUL IN THE CUTANEOUS DERMABRASION TECHNIQUE, ACHIEVED BY MEANS OF ABRASIVE POWDER, as in claim 15, characterised in that the equipment is controlled by an electromechanical or pneumatic valve mechanism (107) acting in synchrony with the valve electromechanical mechanism (108) controlling the pressure mechanism, so that if one is activated the other will remain at rest and vice versa. (Patent 9200599 Priority 18-3-92).

**17.-** MEDICAL EQUIPMENT, USEFUL IN THE CUTANEOUS DERMABRASION TECHNIQUE, ACHIEVED BY MEANS OF ABRASIVE POWDER, as in claims 15 and 16, characterised in that the system has a switch type fott-operated control system (109) taking up three positions, a neutral position in which the control and vacuum systems are idle; another position in which the pressure mechanism is activated, and the Venturi effect is idle, and the third position in which the Venturi effect is activated and the pressure component is idle. (Patent 9200599 Priority 18-3-92).

**18.-** MEDICAL EQUIPMENT, USEFUL IN THE CUTANEOUS DERMABRASION TECHNIQUE, ACHIEVED BY MEANS OF ABRASIVE POWDER, as in claims 1 and 2, characterised in that the filter container (9) fixed to the abrasive powder tank (8) by means of a clamp (68) and an elastic joint (69) is cylindrical in shape, its upper face (60) being provided with a hole (61) communicating with the inside in which a system (57) with two openings is coupled, one opening (62) for the vacuum pump (7) and the other for the conduit pipe (63) from the control mechanism (58) (59); the lower face is provided with an opening system (64) to expedite access to the filters system (9); the side face (65) communicating with the used abrasive powder collection tank (8) expedites the functionality of the suction mechanism (3) and the inside of the tank is provided with a metallic filter (66), another netting type filter (67) being provided surrounding the entire side internal face, whose purpose is to maintain the metallic filter (66) in perfect working order.

**19.-** MEDICAL EQUIPMENT, USEFUL IN THE CUTANEOUS DERMABRASION TECHNIQUE, ACHIEVED BY MEANS OF ABRASIVE POWDER, as in claims 1 to 18, characterised in introducing a shape change in the filter container, which is also an integral part of the apparatus, in the sense that its upper part (203) is cylindrical and its lower part ends in a taper (20) with the vertex pointing down, the said cylindrical part being divided into two compartments, an upper compartment (205) and a lower compartment (206) provided with a hermetic seal, both communicating with each other by one or several holes (207), in which one or several metallic filters (208) are coupled, the number depending on the holes (207) provided. (Patent 9202167 Priority 28-10-92).

**20.-** MEDICAL EQUIPMENT, USEFUL IN THE CUTANEOUS DERMABRASION TECHNIQUE, ACHIEVED BY MEANS OF ABRASIVE POWDER, as in claim 19, characterised in that the upper cylindrical compartment (205) of the filter container has been provided on its upper face with a hole (210) with an outlet to the outside, wherein is coupled a valve control system (211) connecting the pressure component (213) through a path with the suction system (212), its function being to clean the filter system (208) and (214) when the pressure system path (207) is opened, compressed air being passed through such system, and the suction system being at the same time closed. (Patent 9202167 Priority 28-10-92).

**21.-** MEDICAL EQUIPMENT, USEFUL IN THE CUTANEOUS DERMABRASION TECHNIQUE, ACHIEVED BY MEANS OF ABRASIVE POWDER, as in claims 19 and 20, characterised in that the valve system (211) can be manually operated, with a lever system opening a path to expedite the operation of a pressure or suction system, closing the opposite one; electromechanical, activated automatically through a timer or by means of an electric switch, or pneumatically activated. (Patent 9202167 Priority 28-10-92).

**22.-** MEDICAL EQUIPMENT, USEFUL IN THE CUTANEOUS DERMABRASION TECHNIQUE, ACHIEVED BY MEANS OF ABRASIVE POWDER, as in claims 19 to 21, characterised in that the cylindrical portion (206) of the filter container is fitted with a netting type filter (214) and the taper vertex (215) communicates (222) the filter system (202) with the powder and detritus collecting tank (Patent 9202167 Priority 28-10-92).

**23.-** MEDICAL EQUIPMENT, USEFUL IN THE CUTANEOUS DERMABRASION TECHNIQUE, ACHIEVED BY MEANS OF ABRASIVE POWDER, as in claims 1, 12 and 13, characterised in that the used abrasive powder and detritus collecting tank (8) is cylindrical, with a hermetic seal, its upper face (70) having a hole (71) in which the pipe (72) is connected, with its other end communicating with the metallic suction conduit (13); the lower face (73) has a hole (74) communicating with the inside for emptying and a hermetic sealing system in order not to alter the suction component; the inside of the tank is communicated through a small conduit pipe (76) with its inner free end through which the used abrasive powder and the cutaneous detritus are discharged; the side face communicating with the filter container (9) through a communication hole (78) has an angle sheet (79) partially surrounding the hole, its lower part having an upward direction.

**24.-** MEDICAL EQUIPMENT, USEFUL IN THE CUTANEOUS DERMABRASION TECHNIQUE, ACHIEVED BY MEANS OF ABRASIVE POWDER, as in claims 1 to 23, characterised in that the inside of the powder and detritus collecting tank (216) is coupled with a porous material bag (217), such as paper, cloth or other convenient material, with the three-fold function of collecting used powder and detritus, working as a filter and avoiding contagion of the used powder and detritus manipulator. (Patent 9202167 Priority 28-10-92).

**25.-** MEDICAL EQUIPMENT, USEFUL IN THE CUTANEOUS DERMABRASION TECHNIQUE, ACHIEVED BY MEANS OF ABRASIVE POWDER, as in claim 24, characterised in that the powder and detritus collecting tank (216) is cylindrical, with a lower flat face (218), with a hermetic seal (219) to the cylinder body, its upper face (221) having two holes (one of which (22) communicates with the filter system (202) through the vertex of the taper hole (205) and the other (223) through which the outer part of the non-metallic suction conduit (224) leading from the application component is connected. (Patent 9202167 Priority 28-10-92).

**26.-** MEDICAL EQUIPMENT, USEFUL IN THE CUTANEOUS DERMABRASION TECHNIQUE, ACHIEVED BY MEANS OF ABRASIVE POWDER, as in claims 24 and 25, characterised in that the lower face of the hole (223) connects with the connecting conduit (225) of the porous bag (217), smaller in size than the powder and detritus collecting tank. (Patent 9202167 Priority 28-10-92).

**27.-** MEDICAL EQUIPMENT, USEFUL IN THE CUTANEOUS DERMABRASION TECHNIQUE, ACHIEVED BY MEANS OF ABRASIVE POWDER, as in claims 24 to 26, characterised in that the abrasive powder and detritus tank (216) is split into two parts, one being the lower face (218) joined (219) to the cylindrical body (220) and the other being the upper face (222), thereby to expedite the arrangement and removal of the porous bag, both parts of the tank (216) being closed (219) with a thread type seal or clamp system surrounding such tank. (Patent 9202167 Priority 28-10-92).
